# EUROPEAN PATENT APPLICATION

(11) **EP 4 306 655 A1**
(43) Date of publication of application: **17.01.2024**
(21) Application number: 21952232.3
(22) Date of filing: 04.08.2021
(51) Int. Cl.: C12Q 1/6883, C12N 15/11

(54) **PRIMER-PROBE SET, KIT AND DETECTION METHOD FOR DETECTING HUMAN HISTAMINE RECEPTOR HRH1 MRNA**

(71) Applicant: Hangzhou Zheda Dixun Biological Gene Engineering Co., Ltd, Hangzhou, Zhejiang 310052 (CN)
(72) Inventor: WU, Shandong, Hangzhou, Zhejiang 310052 (CN); LIU, Yi, Hangzhou, Zhejiang 310052 (CN); WU, Zhoujie, Hangzhou, Zhejiang 310052 (CN); JIANG, Xuehan, Hangzhou, Zhejiang 310052 (CN); WANG, Jiping, Hangzhou, Zhejiang 310052 (CN); WANG, Meijie, Hangzhou, Zhejiang 310052 (CN); YANG, Xukai, Hangzhou, Zhejiang 310052 (CN)
(74) Representative: Casci, Tamara
(86) International application number: PCT/CN2021/110529
(87) International publication number: WO 2023/010330

(57) **Abstract**

The present disclosure provides a primer probe set for human histamine receptor *HRH1* mRNA detection, a kit and a detection method, and relates to the technical field of biological detection. In the present disclosure, the primer probe set includes a HRH1-F, a HRH1-R and a probe H1-Probe; where the HRH1-F has a nucleotide sequence shown in SEQ ID NO. 1, the HRH1-R has a nucleotide sequence shown in SEQ ID NO. 2, and the probe H1-Probe has a nucleotide sequence shown in SEQ ID NO. 3. The present disclosure provides a kit including the primer probe set and a detection method. An expression level of the *HRH1* mRNA can be detected using an RNA one-step method.

## Description

### TECHNICAL FIELD

The present disclosure belongs to the technical field of biological detection, and specifically relates to a primer probe set for human histamine receptor *HRH1* mRNA detection, a kit and a detection method.

### BACKGROUND ART

Histamine is an active amine compound widely present in animals and plants. The histamine is formed by the decarboxylation of histidine and is usually stored in mast cells of tissues. Histamine is an important chemical conductive substance in the body, and can affect many cellular reactions, including allergies, inflammatory reactions, gastric acid secretion, etc. When the body is stimulated to trigger an antigen-antibody reaction, the cell membrane permeability of mast cells is changed to release the histamine, and the histamine interacts with a histamine receptor to produce pathophysiological effects. The synthesis of a histamine mainly occurs in mast cells, basophils, lungs, skin and gastrointestinal mucosa, and is consistent with the tissues that store the histamine. Histamine, like other transmitters, binds to specific receptors on target cells, thereby changing the biological activity of cells and exerting wide physiological or pathological effects. The inflammatory effect of histamine, namely the effect of histamine on the immune homeostasis in the body depends on the expression and activity of currently-known 4 histamine receptors. The 4 histamine receptors are named in the order of discovery as follows: a histamine 1 (H1) receptor, a histamine 2 (H2) receptor, a histamine 3 (H3) receptor, and a histamine 4 (H4) receptor; where, the H1 receptor is very important in mediating the occurrence of allergic inflammations, including promoting the maturation of dendritic cells and regulating the balance between T helper 1 (Th1) cells/T helper 2 (Th2) cells. The H1 receptor is a member of the rhodopsin-like family of G protein-coupled receptors (GPCR). The H1 receptor is mainly expressed in endothelium, smooth muscle, vascular endothelial cells, heart and central nervous system, and regulate vasodilation and bronchoconstriction. The H1 receptor is a main therapeutic target for allergy. When histamine acts on the H1 receptor, the intracellular cyclic guanosine monophosphate (C-GMP) increases, showing increased heart rate, vasoconstriction, bronchoconstriction, small intestinal smooth muscle contraction, and increased microvascular permeability. Generally, the activated and non-activated forms of the H1 receptor are in an equilibrium state. In allergic diseases, a large amount of immunoglobulins E (IgE) stimulate the mast cells and basophils to release histamine to activate the histamine receptors, leading to symptoms of allergic diseases. The expression level of histamine receptor mRNA in allergic patients is higher than that in healthy people.

H1 antihistamines are widely used in the treatment of allergic diseases such as seasonal and perennial allergic rhinitis, urticaria and atopic dermatitis. The H1 antihistamines exert the antihistamine effect mainly by inversely agonizing an inactive form of the H1 receptor. H1 antihistamines can not only down-regulate the expression level of H1 receptor mRNA induced by histamine, but also down-regulate the basal expression level of H1 receptor and reduce its inherent activity without histamine stimulation.

Most patients with allergic diseases use symptomatic treatment based on the experience of doctors and use antihistamines to relieve allergic symptoms. However, antihistamine therapy is not effective for all patients, and cannot quantify the therapeutic effect. There are no relevant studies or records on this at present.

### SUMMARY

In view of this, the present disclosure provides a primer probe set for human histamine receptor *HRH1* mRNA detection, a kit and use. An expression level of the *HRH1* mRNA can be one-step quantitatively detected. The present disclosure provides a detection method with high accuracy, wide detection range and high sensitivity for the detection of HRH1 proteins.

In order to realize the above objective, the present disclosure provides the following technical solutions:

The present disclosure provides a primer probe set for human histamine receptor *HRH1* mRNA detection, including a HRH1-F, a HRH1-R and a probe H1-Probe; where
the HRH1-F has a nucleotide sequence shown in SEQ ID NO. 1, the HRH1-R has a nucleotide sequence shown in SEQ ID NO. 2, and the probe H1-Probe has a nucleotide sequence shown in SEQ ID NO. 3.

Preferably, the primer probe set may further include a primer pair and a probe of a reference gene *GAPDH,* where the primer pair of the *GAPDH* may include a GAPDH-F and a GAPDH-R, and the probe of the *GAPDH* may include a G-Probe; and
the GAPDH-F may have a nucleotide sequence shown in SEQ ID NO. 4, the GAPDH-R may have a nucleotide sequence shown in SEQ ID NO. 5, and the G-Probe may have a nucleotide sequence shown in SEQ ID NO. ID NO. 6.

Preferably, 5'-ends of the probes H1-Probe and G-Probe may be each labeled with different fluorescent labeling groups, and 3'-ends of the probes H1-Probe and G-Probe may be each labeled with a same quenching group or different quenching groups.

Preferably, the fluorescent labeling group may include a 6-carboxyfluorescein (FAM) or a 2,7-dimethyl-4,5-dichloro-6-carboxyfluorescein (JOE), and the quenching group may include a Black Hole Quencher-1 (BHQ1).

The present disclosure further provides a kit for one-step detection of an expression level of a human histamine receptor *HRH1* mRNA, including a mixed solution of the primer probe set, a PCR reaction solution, an enzyme mixed solution, a human histamine receptor HRH1 standard, an carboxy-X-rhodamine (ROX)reference dye and nuclease-free water.

Preferably, the HRH1-F, the HRH1-R, the probe H1-Probe, the GAPDH-F, the GAPDH-R and the G-Probe in the mixed solution of the primer probe set may have a concentration of 1 nM, 1 nM, 1.5 nM, 2 nM, 2 nM and 3 nM, respectively.

Preferably, the PCR reaction solution may include a deoxy-ribonucleoside triphosphate (dNTP) mix, MgCl₂ and a buffer; and
the enzyme mixed solution may include a thermus aquaticus (Taq) enzyme, a reverse transcriptase, a ribonuclease (RNase) inhibitor and a Taq enzyme antibody with a mass ratio of 15:6:3:1.

The present disclosure further provides a method for one-step detection of an expression level of the human histamine receptor *HRH1* mRNA based on the kit, including the following steps: preparing a reaction system with the kit by using the human histamine receptor HRH1 standard as a template, conducting a quantitative real-time polymerase chain reaction (qRT-PCR), and constructing a standard curve using a logarithm of a copy number as an abscissa and a Ct value as an ordinate;
preparing the same reaction system using an RNA extracted from a sample as a template, conducting the same qRT-PCR, and measuring the expression level of the human histamine receptor *HRH1* mRNA using the standard curve.

Preferably, the reaction system, calculated in 20 µL, may include: 2.4 µL of the nuclease-free water, 10 µL of the PCR reaction solution, 0.5 µL of the enzyme mixed solution, 0.5 µL of the ROX reference dye, 2 µL of the mixed solution of the primer probe set and 5 µL of the template.

Preferably, a qRT-PCR program may include: 42°C for 30 min; 95°C for 1 min; 95°C for 5 s, and 60°C for 31 s, 40 cycles.

Preferably, the standard curve may be y=-3.177x+34.178, R²=0.996.

The present disclosure further provides use of the primer probe set or the kit as a basis in preparing a drug for treating an immune disease or as a tool in dynamically monitoring a therapeutic effect.

Compared with the prior art, the present disclosure has the beneficial effect that: the present disclosure provides a primer probe set for human histamine receptor *HRH1* mRNA detection, and a kit including the primer probe set, which can detect the expression level of the human histamine receptor *HRH1* mRNA by an RNA one-step method. On the one hand, whether the patient's allergic symptoms are caused by the histamine pathway can be determined (allergy symptoms caused by a non-histamine pathway do not express histamine receptors) and the dosage can be guided (a higher H1 receptor expression requires a higher dose of H1 antihistamines). On the other hand, the treatment effect of H1 antihistamines can be dynamically monitored, thereby providing a detection method with high accuracy, wide detection range and high sensitivity for the detection of HRH1 proteins.

In the present disclosure, the primer probes constitute a kit, and one-step detection is conducted based on the kit without separate reverse transcription, which greatly reduces the risk of causing aerosol pollution. Compared with immunological detection methods, the detection method of the present disclosure has high sensitivity, can detect low-concentration clinical samples, can sensitively detect changes in HRH1 content, and has a detection range spanning at least 6 orders of magnitude. Accordingly, the accuracy of the detection results is increased, and at least 80 people can be detected within 1 hour, such that the treatment effect can be dynamically monitored and evaluated in an earlier, more accurate, and faster manner.

### BRIEF DESCRIPTION OF THE DRAWINGS

FIG. 1 is a standard curve of TaqMan real-time fluorescence quantitative RT-PCR for *HRH1* mRNA.
FIG. 2 is a result of precision detection, where 1: 1.0× 10⁷ copies/µL, and 2: 1.0× 10⁴ copies/µL.
FIG. 3 is a result of accuracy detection.
FIG. 4 is a result of sensitivity detection.
FIG. 5 is a result of clinical sample detection, where 1: Case 1 *GAPDH* mRNA before treatment; 2: Case 1 *GAPDH* mRNA after treatment; 3: Case 1 *HRH1* mRNA before treatment; 4: Case 1 *HRH1* mRNA after treatment.
FIG. 6 is a low-precision amplification curve in the case of non-optimal primer and probe designs.
FIG. 7 is an effect of enzyme mixed solution on amplification.
FIG. 8 is a plasmid map of pGM-T vector.

### DETAILED DESCRIPTION OF THE EMBODIMENTS

**The present disclosure will be further described below in conjunction with the examples and accompanying drawings.**

The present disclosure provides a primer probe set for human histamine receptor *HRH1* mRNA detection, including a HRH1-F, a HRH1-R and a probe H1-Probe; where
the HRH1-F has a nucleotide sequence shown in SEQ ID NO. 1, the HRH1-R has a nucleotide sequence shown in SEQ ID NO. 2, and the probe H1-Probe has a nucleotide sequence shown in SEQ ID NO. 3.

In the present disclosure, the primer probe set preferably further includes a primer pair and a probe of a reference gene *GAPDH,* the primer pair of the *GAPDH* preferably includes a GAPDH-F and a GAPDH-R, and the probe of the *GAPDH* preferably includes a G-Probe; the GAPDH-F preferably has a nucleotide sequence shown in SEQ ID NO. 4, the GAPDH-R preferably has a nucleotide sequence shown in SEQ ID NO. 5, and the G-Probe preferably has a nucleotide sequence shown in SEQ ID NO. 6. In the present disclosure, 5'-ends of the probes H1-Probe and G-Probe are each labeled with different fluorescent labeling groups, and 3'-ends of the probes H1-Probe and G-Probe are each labeled with a same quenching group or different quenching groups; and the fluorescent labeling group preferably includes a FAM or a JOE, and the quenching group includes a BHQ1. In an example of the present disclosure, for the probe H1-Probe, the 5'-end is labeled with the FAM, and the 3'-end is labeled with the BHQ1; for the probe G-Probe, the 5'-end is labeled with the JOE, and the 3'-end is labeled with the BHQ1. The present disclosure preferably entrusts Shanghai Sunny Biotechnology Co., Ltd. to synthesize the above primers (Table 1).

**Table 1 TaqMan real-time fluorescence quantitative PCR of primer probe**

| Name | Primer sequence (5'→3') | Amplified fragment |
|---|---|---|
| HRH1-F | AATCCTTCTCTCGAACGGACT | 84 bp |
| HRH1-R | GGCCTGTGTTAGACCCACTC | |
| H1-Probe | (FAM)-TTGCCTTTGCCTGGTGCTGTC-(BHQ1) | |
| GAPDH-F | GACAACAGCCTCAAGATCATC | 70 bp |
| GAPDH-R | CGCCACAGTTTCCCGGAG | |
| G-Probe | (JOE)-ACTCATGACCACAGTCCATGCCAT-(BHQ1) | |

In the present disclosure, the primer probe set when being used is preferably in the form of a mixed solution; and the HRH1-F, the HRH1-R, the probe H1-Probe, the GAPDH-F, the GAPDH-R and the G-Probe in the mixed solution of the primer probe set have a concentration of 1 nM, 1 nM, 1.5 nM, 2 nM, 2 nM and 3 nM, respectively.

The present disclosure further provides a kit for one-step detection of an expression level of a human histamine receptor *HRH1* mRNA, including a mixed solution of the primer probe set, a PCR reaction solution, an enzyme mixed solution, a human histamine receptor HRH1 standard, an ROX reference dye and nuclease-free water.

In the present disclosure, the mixed solution is preferably the same as the above mixed solution and is not further described any more.

In the present disclosure, the PCR reaction solution preferably includes a dNTP mix, MgCl₂ and a buffer; the dNTP mix is preferably a mixture of a dATP, a dTTP, a dCTP, and a dGTP, which is purchased from Thermo Fisher Scientific (product number: R0192), and has a working concentration of preferably 0.1-1 mM; the MgCl₂ has a concentration of preferably 5-20 mM; and the buffer is a 10-50 mM Tris-HCl buffer (at pH 8.0). The enzyme mixed solution preferably includes a Taq enzyme, a reverse transcriptase, an RNase inhibitor and a Taq enzyme antibody with a mass ratio of preferably 15:6:3:1 to obtain the best amplification efficiency. The histamine receptor HRH1 standard is preferably an RNA standard for preparing a standard curve.

In the present disclosure, the Taq enzyme is a heat-resistant Taq DNA polymerase, deoxynucleotides in the dNTP are added to a 3-OH terminus one by one using the 3'→5' polymerase activity of the Taq enzyme and using DNA as a template; meanwhile, mismatched primer ends can be identified and eliminated using the 5'→3' exonuclease activity of the Taq enzyme, which is related to the correction function during the replication process, nucleotides can also be hydrolyzed from the 5'-end and mismatched nucleotides can also be excised through several nucleotides. In this way, the chain replacement is realized during the chain extension, and the replaced probe is cut off. The reverse transcriptase can reverse transcribe a mRNA into a cDNA for PCR reaction. The RNase inhibitor is used to suppress the activity of an exogenous RNase. The Taq enzyme antibody is an anti-Taq antibody for hot-start PCR, inhibits DNA polymerase activity after binding to the Taq enzyme, and can effectively suppress the non-specific annealing of primers and the non-specific amplification caused by primer dimers under low temperature conditions. Taq enzyme antibody is denatured during the initial DNA denaturation of the PCR reaction, and the Taq enzyme recovers the activity to realize PCR amplification.

The present disclosure further provides a method for one-step detection of the expression level of the human histamine receptor *HRH1* mRNA based on the kit, including the following steps: preparing a reaction system with the kit by using the human histamine receptor HRH1 standard as a template, conducting a quantitative real-time polymerase chain reaction (qRT-PCR), and constructing a standard curve using a logarithm of a copy number as an abscissa and a Ct value as an ordinate;
preparing the same reaction system using an RNA extracted from a sample as a template, conducting the same qRT-PCR, and measuring the expression level of the human histamine receptor *HRH1* mRNA using the standard curve.

In the present disclosure, the reaction system, calculated in 20 µL, preferably includes: 2.4 µL of the nuclease-free water, 10 µL of the PCR reaction solution, 0.5 µL of the enzyme mixed solution, 0.5 µL of the ROX reference dye, 2 µL of the mixed solution of the primer probe set and 5 µL of the standard or a to-be-tested RNA sample. The qRT-PCR program preferably includes: 42°C for 30 min; 95°C for 1 min; 95°C for 5 s, and 60°C for 31 s, 40 cycles. The standard curve is constructed using a logarithm of a copy number as an abscissa (x) and a Ct value as an ordinate (y): y=-3.422x + 37.235 (R²=1.000). There is no special limitation on the source of the RNA, and the RNA can be extracted from human blood, nasal secretions, bronchial irrigating fluid, saliva or tear samples.

The present disclosure further provides use of the primer probe set or the kit as a basis in preparing a drug for treating an immune disease or as a tool in dynamically monitoring a therapeutic effect.

In the present disclosure, the use is preferably the same as the above method and is not further described any more.

The primer probe set for human histamine receptor *HRH1* mRNA detection, the kit and the detection method provided by the present disclosure are described in detail below with reference to the examples, but these examples should not be understood as limiting the claimed protection scope of the present disclosure.

Unless otherwise specified, the reagents, kits and instruments used in the present disclosure are all commercially-available conventional in the art:
a whole-blood total RNA kit (Hangzhou Simgen Biological Reagent Development Co., Ltd., product number: 5201050);
a HiScribe T7 High Yield RNA Synthesis Kit (New England Biolabs, product number: E2050S);
an Applied Biosystems^{™} 7300 fluorescence quantitative PCR instrument (Thermo Fisher Scientific, USA);
a -80°C low-temperature refrigerator (Thermo Fisher Scientific, USA);
a high-speed and low-temperature table centrifuge (Eppendorf, Germany); and
a Qubit 3 Fluorometer (Thermo Fisher Scientific, USA).

### Example 1

1. Shanghai Sunny Biotechnology Co., Ltd. was entrusted to synthesize the primers and probes shown in Table 1.
2. Preparation of a standard

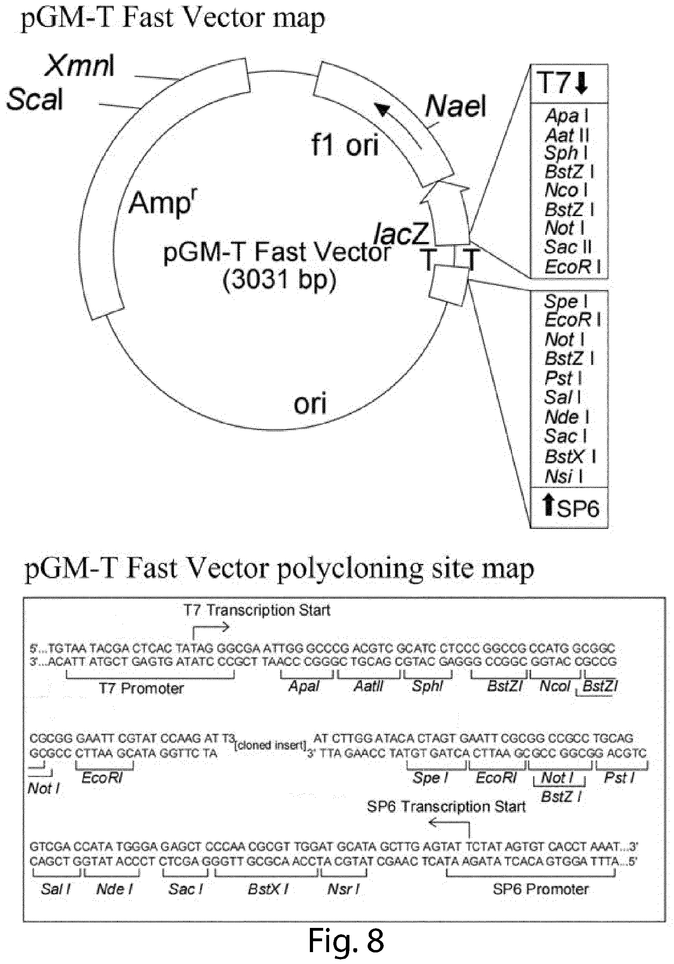

In-vitro transcription: a pGM-T ligation kit [TIANGEN Biotech (Beijing) Co., Ltd., product number: VT202-01] was used, a HRH1 plasmid DNA (constructed and synthesized by entrusting Nanjing GenScript Biotech Co., Ltd., FIG. 8) was constructed using a pGM-T as a vector, and HRH1 plasmid DNA was transcribed into a mRNA in vitro using the HiScribe T7 High Yield RNA Synthesis Kit (NEW ENGLAND BioLabs, product number: E2040S).

The initial copy number of RNA was calculated according to a copy number calculation formula: copy number=[6.02×10²³×RNA concentration (ng/µL)×10⁻⁹]/[RNA length (bp)×340]. The *HRH1* mRNA was diluted with nuclease-free water to 1.0×10¹⁰ copies/µL, to obtain a *HRH1* mRNA standard.

3. Extraction and dilution of whole-blood RNA: whole-blood total RNA was extracted from ethylenediaminetetraacetic acid (EDTA) anticoagulated whole-blood samples with the whole-blood total RNA kit, quantificated with the Qubit 3 fluorometer, and diluted with the nuclease-free water to 20 ng/µL.

### 4. TaqMan real-time fluorescent quantitative PCR

A 20 µL system was prepared using the standard/whole-blood RNA as a template with: 2.4 µL of the nuclease-free water, 10 µL of the PCR reaction solution, 0.5 µL of the enzyme mixed solution, 0.5 µL of the ROX reference dye, 2 µL of the mixed solution of the primer probe set and 5 µL of the standard or a to-be-tested RNA sample.

A qRT-PCR program was as follow: 42°C for 30 min; 95°C for 1 min; 95°C for 5 s, and 60°C for 31 s, 40 cycles. A detection fluorescein was set up: FAM, JOE; a reference fluorescence was: ROX; reaction system was: 20 µL; and the fluorescence signal collection was: 60°C for 31 sec.

### 5. Generation of a standard curve

The HRH1 standard was diluted in a 10-fold gradient using 1.0×10⁸-1.0×10³ copies/µL as a template, 3 replicates were conducted for each dilution, and TaqMan real-time fluorescence quantitative RT-PCR detection was conducted to generate a standard curve.

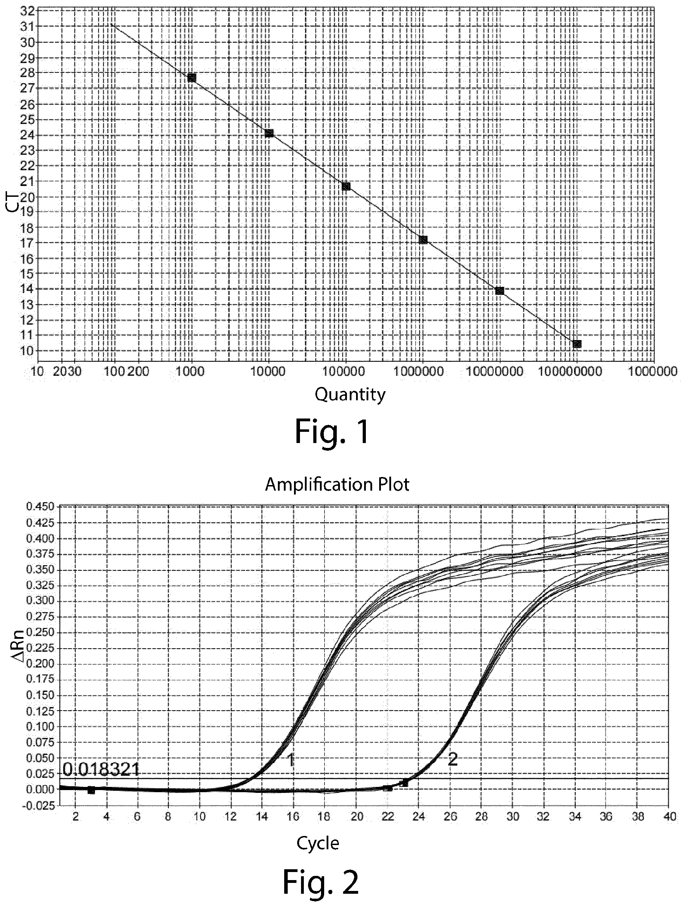

The result is shown in FIG. 1. The logarithm of the copy number is taken as the abscissa (x) and the Ct value is taken as the ordinate (y), and a regression equation is obtained: y=-3.422x+37.235 (R²=1.000), indicating the logarithm of the copy number of the standard equation has a very high correlation with the Ct value.

### 6. Precision detection

Standards of 1.0×10⁷ copies/µL and 1.0×10⁴ copies/µL were taken as templates, 10 replicates were conducted for each concentration; 10 times of TaqMan real-time fluorescent quantitative RT-PCR detections were conducted, a coefficient of variation of the logarithm of each concentration was calculated, respectively; and a statistical analysis was conducted to analyze the precision of the detection method.

The results are shown in Table 2 and FIG. 2. The coefficient of variation of the logarithm of each concentration is 0.380% and 0.539% separately, and is less than 5%, indicating that the TaqMan real-time fluorescent quantitative RT-PCR detection method established by the present disclosure has excellent precision.

**Table 2 Precision detection result**

| Theoretical copy number | Mean logarithm of copy number | SD | C.V |
|---|---|---|---|
| 1.0×10⁷ | 6.976 | 0.027 | 0.380% |
| 1.0×10⁴ | 4.003 | 0.022 | 0.539% |

### 7. Accuracy detection

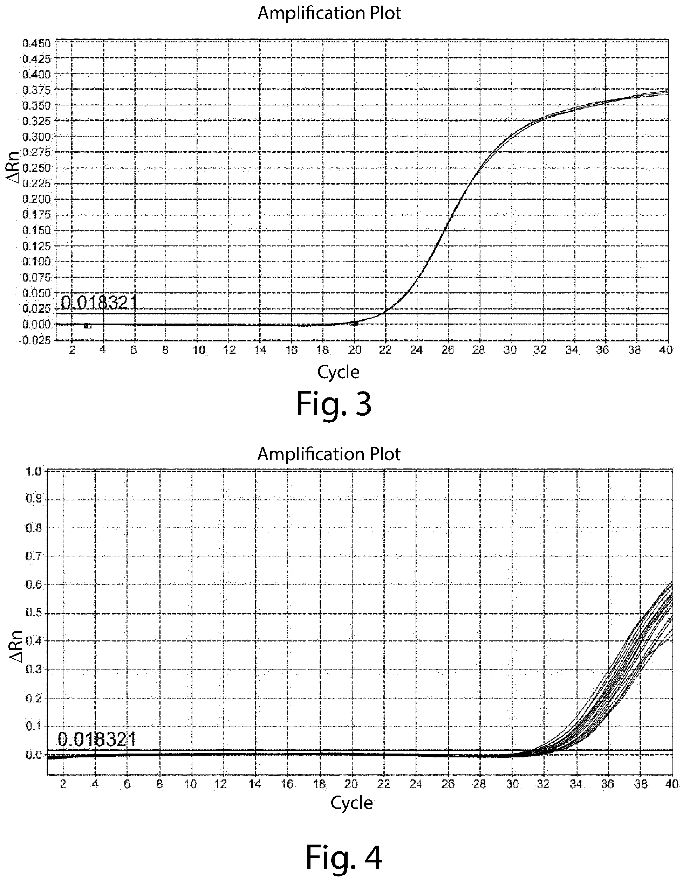

A 1.0×10⁶ copies/µL standard was subjected to a 30-time dilution (2 µL 1.0×10⁶ copies/µL standard + 58 µL nuclease-free water) as a template, for 3 replicates; 3 times of TaqMan real-time fluorescence quantitative RT-PCR detections were conducted, and an absolute deviation of the logarithm of each concentration was calculated. The results are shown in FIG. 3. The absolute deviation of the logarithm of each concentration is 0.013, -0.004, and -0.010, respectively, within the range of ±0.5, indicating that the TaqMan real-time fluorescent quantitative RT-PCR detection method established by the present disclosure has excellent accuracy.

**Table 3 Accuracy detection data**

| C_{T} | Results (copies/µL) | Copy number logarithm | Theoretical copy number (copies/µL) | Theoretical copy number logarithm | Absolute deviation |
|---|---|---|---|---|---|
| 21.713 | 3.434×10⁴ | 4.536 | 3.333×10⁴ | 4.523 | 0.013 |
| 21.771 | 3.302×10⁴ | 4.519 | | | -0.004 |
| 21.790 | 3.259×10⁴ | 4.513 | | | -0.010 |

8. Sensitivity detection

A 10.0 copies/µL standard was taken as a template, for 25 replicates, 25 times of TaqMan real-time fluorescence quantitative RT-PCR detection were conducted to check whether there were amplifications, and the sensitivity of the detection method was analyzed.

The results are shown in Table 4 and FIG. 4. A total of 25 detection results are obtained, reaching 100%. This indicates that the TaqMan real-time fluorescent quantitative RT-PCR detection method established by the present disclosure has very high sensitivity, and the minimum number of detected copies is less than 10 copies/ µL.

**Table 4 Ct value results of sensitivity detection**

| | | | | |
|---|---|---|---|---|
| 32.435 | 32.450 | 32.998 | 33.082 | 31.590 |
| 32.149 | 31.957 | 32.612 | 32.142 | 31.225 |
| 31.892 | 32.739 | 32.496 | 32.059 | 32.104 |
| 31.829 | 32.396 | 32.925 | 32.076 | 31.819 |
| 33.021 | 31.887 | 32.168 | 32.670 | 31.634 |

### 9. Clinical sample detection

Whole-blood samples of patients and normal persons were taken to extract and dilute whole-blood RNA according to the above steps, and TaqMan real-time fluorescent quantitative RT-PCR detection was conducted according to the above steps.

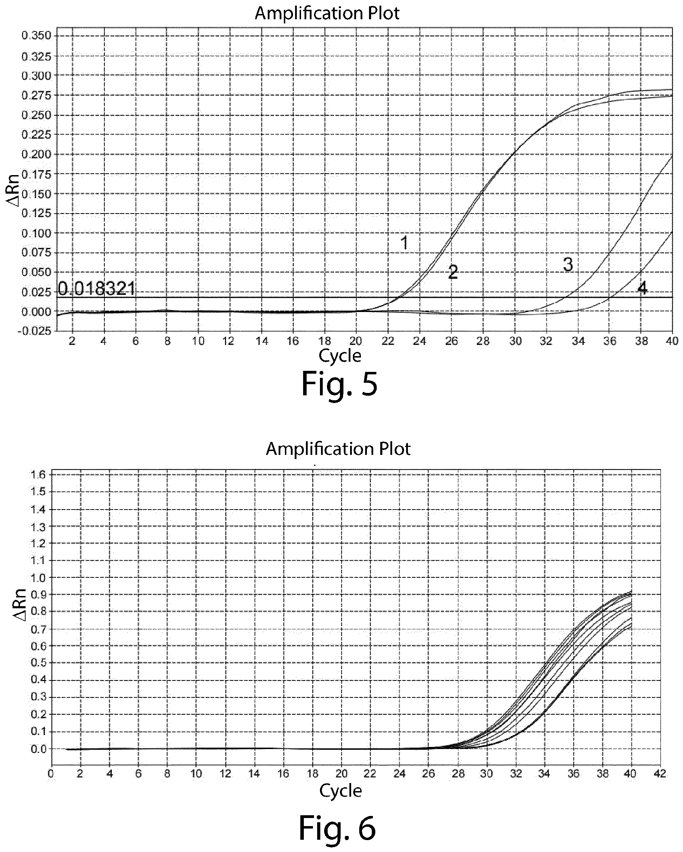

Compared with a domestic brand of human histamine H1 receptor (HRH1) detection kit (enzyme-linked immunosorbent assay), the results are shown in Table 5 and FIG. 5. The TaqMan real-time fluorescent quantitative RT-PCR detection method established in the present disclosure has better sensitivity and specificity than that of a counterpart reagent, and can effectively monitor the treatment effect.

**Table 5 Comparison of clinical sample detection experiments**

| SN | Sample type | Product of the present disclosure | | Human histamine H1 receptor (HRH1) detection kit (enzyme-linked immunosorbent assay) | |
|---|---|---|---|---|---|
| | | Results (copies/µL) | Positive/negative | Results (ng/mL) | Positive/negative |
| **1** | Case 1 before treatment | 74.407 | + | 156.7 | + |
| **2** | Case 1 after treatment | 11.480 | - | 89.3 | + |
| **3** | Case 2 before treatment | 84.491 | + | 267.9 | + |
| **4** | Case 2 after treatment | 1.479 | - | 49.7 | - |
| **5** | Case 3 before treatment | 89.283 | + | 187.6 | + |
| **6** | Case 3 after treatment | 1.017 | - | 42.7 | - |
| **7** | Case 4 | 29.012 | + | 56.1 | - |
| **8** | Healthy control 1 | 3.964 | - | 32.7 | - |
| **9** | Healthy control 2 | 3.027 | - | 67.9 | + |
| 10 | Healthy control 3 | 2.244 | - | 47.6 | - |

### Comparative Example 1 Results of amplification using other non-optimal primers and probes

The primers and probes in the system used in the present disclosure in Example 6 were replaced with other non-optimal primers and probes. The results are shown in Table 6 and FIG. 6. A coefficient of variation of the logarithm of the low-precision concentration exceeds 5%, reaching 8.006%.

Sequences of non-optimal HRH1 primers and probes were used:
HRH1-F (SEQ ID NO.7): CAGGGACTATGTAGCCGTCA;
HRH1-R (SEQ ID NO.8): AGAGAAGGATTGGCTATCACC; and
H1-Probe (SEQ ID NO.9): (FAM)-CTGATATCTCGCTGGCCCCATG-(BHQ1).

**Table 6 Amplification of non-optimal primers and probes**

| Theoretical copy number | Mean logarithm of copy number | SD | C.V |
|---|---|---|---|
| 1.0×10⁴ | 3.657 | 0.293 | 8.006% |

### Comparative Example 2 Comparison of the effect of enzyme mixed solution

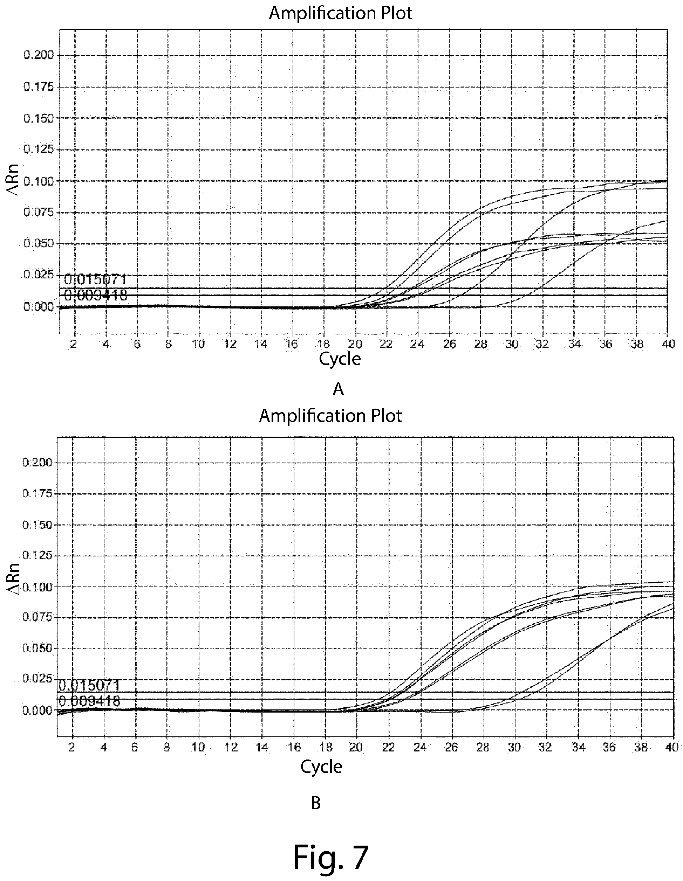

An amplification was conducted on 4 cases of whole-blood RNA samples using a non-optimal ratio of enzyme mixed solution (the Taq enzyme, reverse transcriptase, RNase inhibitor and Taq enzyme antibody had a mass ratio of 13:7:4:1) and a best ratio of enzyme mixed solution. An amplification result using the non-optimal ratio of enzyme mixed solution is shown in FIG. 7 A, and an amplification result using the best ratio of enzyme mixed solution is shown in FIG. 7 B. It can be seen that the best enzyme mixed solution has better repeatability and better amplification effect.
The above descriptions are merely preferred implementations of the present disclosure. It should be noted that a person of ordinary skill in the art may further make several improvements and modifications without departing from the principle of the present disclosure, but such improvements and modifications should be deemed as falling within the protection scope of the present disclosure.

## Claims

1. A primer probe set for human histamine receptor *HRH1* mRNA detection, comprising a HRH1-F, a HRH1-R and a probe H1-Probe; wherein
the HRH1-F has a nucleotide sequence shown in SEQ ID NO. 1, the HRH1-R has a nucleotide sequence shown in SEQ ID NO. 2, and the probe H1-Probe has a nucleotide sequence shown in SEQ ID NO. 3.

2. The primer probe set according to claim 1, further comprising a primer pair and a probe of a reference gene *GAPDH,* wherein the primer pair of the *GAPDH* comprises a GAPDH-F and a GAPDH-R, and the probe of the *GAPDH* comprises a G-Probe; and
the GAPDH-F has a nucleotide sequence shown in SEQ ID NO. 4, the GAPDH-R has a nucleotide sequence shown in SEQ ID NO. 5, and the G-Probe has a nucleotide sequence shown in SEQ ID NO. ID NO. 6.

3. The primer probe set according to claim 1 or 2, wherein 5'-ends of the probes H1-Probe and G-Probe are each labeled with different fluorescent labeling groups, and 3'-ends of the probes H1-Probe and G-Probe are each labeled with a same quenching group or different quenching groups.

4. The primer probe set according to claim 3, wherein the fluorescent labeling group comprises a 6-carboxyfluorescein (FAM) or a 2,7-dimethyl-4,5-dichloro-6-carboxyfluorescein (JOE), and the quenching group comprises a Black Hole Quencher-1 (BHQ1).

5. A kit for one-step detection of an expression level of a human histamine receptor *HRH1* mRNA, comprising a mixed solution of the primer probe set according to any one of claims 1-4, a PCR reaction solution, an enzyme mixed solution, a human histamine receptor HRH1 standard, an carboxy-X-rhodamine (ROX) reference dye and nuclease-free water.

6. The kit according to claim 5, wherein the HRH1-F, the HRH1-R, the probe H1-Probe, the GAPDH-F, the GAPDH-R and the G-Probe in the mixed solution of the primer probe set have a concentration of 1 nM, 1 nM, 1.5 nM, 2 nM, 2 nM and 3 nM, respectively.

7. The kit according to claim 5, wherein the PCR reaction solution comprises a deoxy-ribonucleoside triphosphate (dNTP) mix, MgCl₂ and a buffer; and
the enzyme mixed solution comprises a thermus aquaticus (Taq) enzyme, a reverse transcriptase, a ribonuclease (RNase) inhibitor and a Taq enzyme antibody with a mass ratio of 15:6:3:1.

8. A method for detecting an expression level of a human histamine receptor *HRH1* mRNA in one step based on the kit according to any one of claims 5-7, comprising the following steps:
preparing a same reaction system using an RNA extracted from a sample as a template, conducting a same quantitative real-time polymerase chain reaction (qRT-PCR), and determining the expression level of the human histamine receptor *HRH1* mRNA using a standard curve constructed by an *HRH1* mRNA standard.

9. The method according to claim 8, wherein a process for constructing the standard curve comprises: preparing a reaction system with the kit and using the human histamine receptor *HRH1* mRNA standard as a template, conducting a qRT-PCR, and constructing the standard curve using a logarithmic value of a copy number as an abscissa and a Ct value as an ordinate.

10. The method according to claim 8 or 9, wherein the reaction system is 20 µl, comprising: 2.4 µl of the nuclease-free water, 10 µl of the PCR reaction solution, 0.5 µl of the enzyme mixed solution, 0.5 µl of the ROX reference dye, 2 µl of the mixed solution of the primer probe set and 5 µl of the template.

11. The method according to claim 8 or 9, wherein a qRT-PCR program comprises: 42°C for 30 min; 95°C for 1 min; 95°C for 5 s, 60°C for 31 s, 40 cycles.

12. The method according to claim 8 or 9, wherein the standard curve is y=-3.177x+34.178, R²=0.996.

13. Use of the primer probe set according to any one of claims 1-4 or the kit according to any one of claims 5-7 as a basis for the preparation of a medicine for treating immune diseases or as a tool for dynamically monitoring therapeutic effects.
